# EUROPÄISCHE PATENTSCHRIFT

(11) **EP 2 809 664 B1**
(45) Veröffentlichungstag und Bekanntmachung des Hinweises auf die Patenterteilung: **30.12.2015**
(21) Anmeldenummer: 13703751.1
(22) Anmeldetag: 30.01.2013
(51) Int. Cl.: C07D 317/12

(54) **VERFAHREN ZUR HERSTELLUNG VON METHYLEN-1,3-DIOXOLANEN**
METHOD FOR PRODUCING METHYLENE-1,3-DIOXOLANES
PROCÉDÉ DE PRODUCTION DE MÉTHYLÈNE-1,3-DIOXOLANES

(30) Priorität: 01.02.2012 EP 12153494
(43) Veröffentlichungstag der Anmeldung: 10.12.2014
(73) Patentinhaber: Bayer Intellectual Property GmbH, 40789 Monheim (DE)
(72) Erfinder: PAZENOK, Sergii, 42699 Solingen (DE); SCHNATTERER, Albert, 51375 Leverkusen (DE)
(74) Vertreter: BIP Patents
(86) Internationale Anmeldenummer: PCT/EP2013/051756
(87) Internationale Veröffentlichungsnummer: WO 2013/113738

(56) Entgegenhaltungen:
- DE-A1- 2 534 851
- JP-A- 2001 302 554
- Jochen Mattay ET AL: "Fragmentierung cyclischer Carboxonium-Ionen, IV Synthese von [beta]-Oxa-[gamma],[delta]-enonen", Liebigs Annalen der Chemie, 1. Januar 1981 (1981-01-01), Seiten 1105-1117, XP055023167, Weinheim DOI: 10.1002/jlac.198119810616 Gefunden im Internet: URL:http://onlinelibrary.wiley.com/store/1 0.1002/jlac.198119810616/asset/19811981061 6_ftp.pdf?v=1&t=h0bz892q&s=34205fc9ce66a08 f71175d3e3b3f93dcb921715f [gefunden am 2012-03-28]

## Beschreibung

Die vorliegende Erfindung betrifft ein neues Verfahren zur Herstellung von Methylen-1,3-dioxolanen. Methylen-1,3-dioxolane sind wichtige Zwischenprodukte zur Herstellung von Pyrazolen und Anthranilsäureamiden, die als Insektizide Verwendung finden können.

In der Literatur ist bereits beschrieben, dass 2-Methylendioxolane aus 2-Chlormethylendioxolanen und KOH hergestellt werden können. So wird in Journal of Polymer Science 1964, vol. 2 p.3471 und Biochemical preparation, 1960 v. 7, 45 berichtet, dass die Umsetzung von 4-Chlormethylen-1,3-dioxolanen mit festem KOH lediglich ca 52 % Ausbeute des Produktes (4-Methylen-1,3-dioxolane) und ca. 60 % Ausbeute an 2,2-Dimethyl-4-methylen-1,3-dioxolan liefert. Gevorkyan et al, Khimiya Geterocycl. Soed. N 1, 1991, pp. 33-36 berichtet dagegen, dass KOH, wenn es als Feststoff oder unter Phasen-Transferbedingungen eingesetzt wird, nicht geeignet ist für die Dehydrochlorierung von 2-Chlormethyldioxolanen. So beschreibt *Gevorkyan et al,* dass eine Isomerisierung unter Verschiebung der Doppelbindung im Ring stattfindet. Die Abspaltung von HCl mit Hilfe von wasserfreiem Natriumdiethylenglycolat (hergestellt aus Na und Diethylenglykol) wurde ebenfalls von Gevorkyan et al, Khimiya Geterocycl. Soed.N 12, 1983, 1607-1613 vorgeschlagen, wobei Ausbeuten von ca. 70-80 % erreicht werden können. Die Verwendung von metallischem Natrium für die industrielle Anwendung ist aus Sicherheitsgründen nachteilig. Während der HCl Abspaltung reagiert ein Teil der entsprechenden Chlormethylendioxolane unter Substitution des Chloratoms durch Ethylenglykolanion, wobei eine hochsiedende Verbindung entsteht. In J. Org. Chem, 1987, 52, 2625-27 wird berichtet, dass 2,2-Dimethyl-4-methylene-1,3-dioxolan nur eine begrenzte Stabilität bei -10°C hat. Dies deutet darauf hin, dass die Stabilität des Produktes vom Herstellungsverfahren abhängig ist.

Mattay et al, Liebigs ann. Chem 1981, 1105-1117 berichten eine Ausbeute von 60-70% unter Verwendung von KOH in Ethylenglykol.

Bei den in der Literatur beschriebenen Verfahren wurden weitere Nachteile beobachtet. So verunmöglicht die Anwendung von NaOH oder KOH als Feststoff im Überschuß ohne Lösungsmittel die Rührbarkeit des Reaktionsgemisches, insbesondere gegen Ende der Reaktion, nachdem das Produkt teils abdestilliert ist. Zusätzlich wurde bei 2-Methylen-4,4-dioxolan beobachtet, dass eine Isomerisierung stattfindet, insbesondere beim Scale up des Prozesses, wobei bis zu 5% des entsprechenden Isomers entstehen können. Außerdem zersetzt sich unter Reaktionsbedingungen ein Teil des Produktes unter Bildung von Aceton. Dies beeinflusst insbesondere beim der Scale up signifikant die Reinheit des Produktes, welches Reste an Aceton enthält. Beobachtet wurden bis zu 4 % Aceton.

Aufgabe der vorliegenden Anmeldung ist daher die Bereitstellung eines neuen Verfahrens zur Herstellung von Methylen-1,3-dioxolanen in guter Reinheit und Ausbeute, welches die bei den in der Literatur beschriebenen Verfahren beobachteten Nachteile nicht aufweist.

Erfindungsgemäß gelöst wurde die Aufgabe durch ein Verfahren zur Herstellung von Methylen-1,3-dioxolanen der allgemeinen Formel (I), 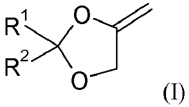 in welcher
R¹ und R² unabhängig voneinander für Wasserstoff, Alkyl, Aryl oder Alkylaryl stehen,
R¹ und R² weiterhin gemeinsam mit dem C-Atom, an das sie geknüpft sind, einen 4 bis 7 gliedrigen, gesättigten, gegebenenfalls substituierten Ring ausbilden können,
indem man Verbindungen der Formel (II), 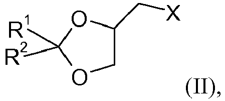 in welcher
- R¹, R²: die oben angegebenen Bedeutungen haben,
- X: für Halogen steht,
mit anorganischen Basen in Gegenwart von Polyethylenglykoldimethylethern oder Polyethylenglykoldiethylethern umsetzt.

Es ist als überraschend anzusehen, dass sich durch dass erfindungsgemäße Verfahren die Methylen-1,3-dioxolane der Formel (I) selektiv und in hoher Ausbeute herstellen lassen, ohne dass störende Nebenreaktionen, wie Ringöffnung, Isomerisierung oder Substitution zu beobachten sind.

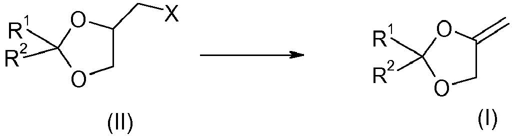

Beispiele für nach dem erfindungsgemäßen Verfahren herstellbare Methylen-1,3-dioxolane der Formel (I) sind:
2,2-Dimethyl-4-methylene-1,3-dioxolan, 4-Methylene-1,3-dioxolan, 2,2-Diethyl-4-methylene-1,3-dioxolan, 2,2-Pentamethylen-4-methylen-1,3-dioxolan, 2,2-Hexamethylen-4-methylen-1,3-dioxolan, 2-Phenyl-4-methylene-1,3-dioxolan, 2-Methyl-4-methylene-1,3-dioxolan.

### Allgemeine Definitionen

Im Zusammenhang mit der vorliegenden Erfindung, umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomere, wie auch die threo- und erythro-, und auch die optischen Isomere, beliebige Mischungen dieser Isomere, sowie die möglichen tautomeren Formen offenbart und beansprucht.

### 4-Halogenmethyl-1,3 -dioxolan-Derivate der Formel (II)

Die bei der Durchführung des erfindungsgemäßen Verfahrens als Ausgangsstoffe verwendeten 4-Halogenmethyl-1,3-dioxolane werden durch die Formel (II) allgemein definiert. 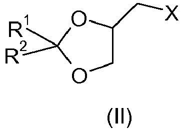 wobei
R¹ und R² unabhängig voneinander für Wasserstoff, Alkyl, Aryl oder Alkylaryl stehen,
R¹ und R² weiterhin gemeinsam mit dem C-Atom, an das sie geknüpft sind, einen 4 bis 7 gliedrigen, gesättigten, gegebenenfalls substituierten Ring ausbilden können,
R¹ und R² unabhängig voneinander bevorzugt für Wasserstoff, (C₁-C₁₂)-Alkyl, Pentamethylen oder Hexamethylen stehen,
R¹ und R² besonders bevorzugt für Wasserstoff, Methyl, Pentamethylen oder Hexamethylen stehen,
X für Halogen steht, bevorzugt für Chlor oder Brom steht, besonders bevorzugt für Chlor steht.

Die Verbindungen sind bekannt und können nach den Verfahren wie in Journal of Polymer Science 1964, vol. 2 p.3471; Biochemical preparation, 1960 v. 7, 45 und durch Gevorkyan et al, Khimiya Geterocycl. Soed. N 1, 1991, pp. 37-39 beschrieben, hergestellt werden.

Beispiele für erfindungsgemäß geeignete Ausgangsstoffe sind 4-Chlormethyl-2,2-dimethyl-1,3-dioxolan, 4-Chlormethyl-1,3-dioxolan, 4-(Chlormethyl)-2-methyl-1,3-dioxolan, 2-(Chlormethyl)-1,4-dioxaspiro[4.4]nonan, 2-(Chlormethyl)-1,4-dioxaspiro[4.5]decan, 4-(Chlormethyl)-2-phenyl-1,3-dioxolan, 4-Brommethyl-2,2-dimethyl-1,3-dioxolan.

### Reaktionsdurchführung

Die Durchführung des erfindungsgemäßen Verfahrensschritts erfolgt vorzugsweise innerhalb eines Temperaturbereichs von 60°C bis 150°C, besonders bevorzugt bei Temperaturen von 80°C bis 140 °C. Der erfindungsgemäße Verfahrensschritt wird im Allgemeinen unter Normaldruck oder im Vakuum durchgeführt.

Die Reaktionszeit ist nicht kritisch und kann, in Abhängigkeit von der Ansatzgröße und Temperatur, in einem Bereich zwischen 1 Std. und mehreren Stunden gewählt werden.

Bei der Durchführung des erfindungsgemäßen Verfahrensschritts setzt man 1 Mol des 4-Halogenmethyl-1,3-dioxolans der Formel (II) mit 0,8 Mol bis 2,5 Mol, vorzugsweise 1 Mol bis 2 Mol, einer anorganischen Base um. Geeignete Basen sind NaOH, KOH, NaOtBut, KOtBut, NaOMe, KOMe. Besonders bevorzugt sind NaOH und KOH, ganz besonders bevorzugt ist NaOH.

Die Reaktion wird in einem Lösungsmittel durchgeführt.

Geeignet sind Polyethylenglykoldimethylether oder Polyethylenglykoldiethylether mit einer Molmasse von 200 bis 500. Besonders bevorzugt sind Polyethylenglykoldimethylether mit einer Molmasse von 250.

Die Aufarbeitung des Reaktionsgemisches erfolgt wasserfrei durch Destillation des Produktes. Vorzugsweise wird das Produkt direkt aus dem Reaktionsgemisch abdestilliert. Die Destillation kann auch im Vakuum erfolgen, um eine zusätzliche thermische Belastung des Produktes zu vermeiden. Der Rückstand (Sump) kann nach der Befreiung von Salzen, beispielsweise von NaCl, wieder eingesetzt werden.

Desweiteren ist auch eine wässrige Aufarbeitung möglich.

Die Reinheit der so erhaltenen Verbindungen der Formel (I) ist sehr hoch und liegt im Bereich von 97 %-99 %, welche ohne Reinigungsschritt weiter eingesetzt werden können. Insbesondere zeichnet sich die erfindungsgemäße Umsetzung durch den Einsatz günstiger Rohstoffe, sowie durch eine auch im großtechnischen Maßstab besonders gut und einfach durchzuführende Prozeßführung aus.

Die Verbindungen der allgemeinen Formel (I) sind wertvolle Zwischenprodukte in der Synthese von Pyrazolsäuren, welche wiederum wichtige Bausteine für die Herstellung von insektizid wirksamen Anthranilsäureamiden (WO2007/112893, WO2007/144100) darstellen. Gemäß dem Schema (I) können die Verbindungen der Formel (I) beispielsweise zu Pyrazolcarbonsäuren umgesetzt werden. 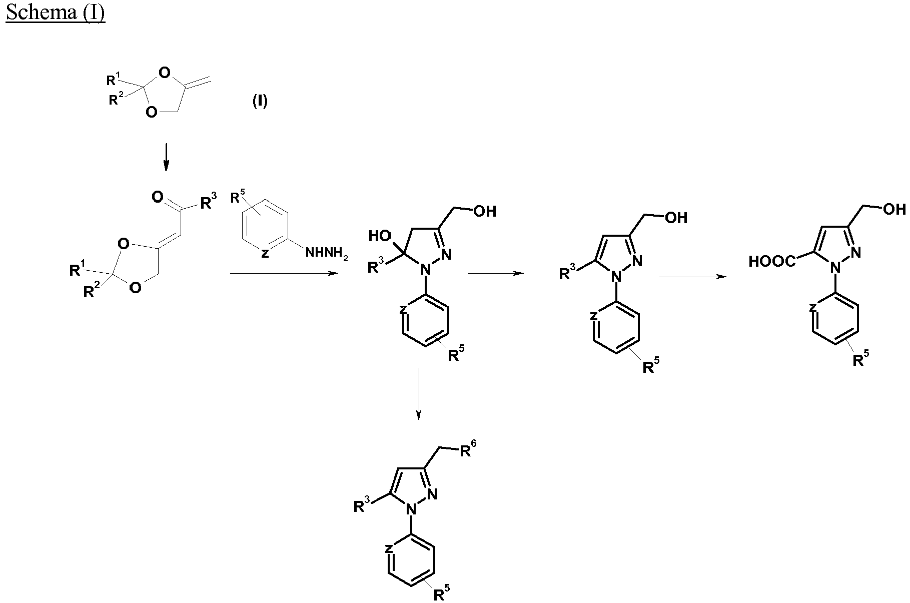 wobei
- R¹, R²: die oben angegebenen Bedeutungen haben,
- R³: für CX₃, (C=O)OAlkyl oder (C=O)OAryl steht,
- X: für Halogen steht,
- R⁵: für Halogen, Cyano, Nitro, Alkyl, Cycloalkyl, Halogenalkyl, Halogencycloalkyl, Alkoxy, Halogenalkoxy, Alkylamino, Dialkylamino, Cycloalkylamino steht,
- R⁶: für Halogen, OSO₂Me, O(C=O)CH₃ steht,
- Z: für CH, N steht.

### Herstellbeispiele

Die folgenden Herstellbeispiele illustrieren die Erfindung, ohne sie zu beschränken.

### Beispiel 1

### 2,2-Dimethyl-4-methylen-1,3-dioxolan

4-Chlormethyl-2,2-dimethyl-1,3-dioxolan (150 g, 1 mol) und 80 g NaOH in 500 ml Polyethylenglykoldimethylether 250 wurden 2 Std. bei 120°C gerührt. Nach 2 Std zeigte die GC Probe vollständigen Umsatz. Vakuum von 100 mbar wurde angelegt und das Produkt wurde in eine gekühlte Vorlage abdestilliert. Man erhielt 125 g (92 %) des Produktes mit der Reinheit von 99 %.

Sp. 103-105°C.

Analytische Charakterisierung :
¹H NMR (CDCl₃) δ: 1,43 (s,6H), 3,81 und 4,20 (dd, 2H), 4,7 (dd, 2H) ppm.

### Beispiel 2

### 4-Methylen-1,3-dioxolan

Man arbeitet wie in Beispiel 1 beschrieben, verwendet jedoch 4-Chlormethyl-1,3-dioxolan.

Die Ausbeute beträgt 87%. Sdp. 72-74 °C . n_{d}²⁰ 1,4372 (siehe Journal of Polymer Science 1964, vol. 2 p.3487*)*.

### Beispiel 3

### 2,2-Pentamethylen-4-methylen-1,3-dioxolan

Man arbeitet wie in Beispiel 1 beschrieben, verwendet jedoch 4-Chlormethyl-2,2 (pentamethylen)-1,3-dioxolan.

Ausbeute 87 % ; Sp: 110-112°C /50 mbar.

Analytische Charakterisierung :
¹H NMR (CDCl₃) δ: 1,35-1,80 (m, 10 H), 4.12 (br.s, 2H), 4,37-4,62 (m,2H) ppm.

### Beispiel 4

### 2-Methyl-4-methylen-1,3-dioxolan

Man arbeitet wie in Beispiel 1 beschrieben, verwendet jedoch 4-Chlormethyl-2-methyl-1,3-dioxolan.

Ausbeute 91 %, S.p. 98-100°C.

Analytische Charakterisierung :
¹H NMR: δ 1,21 (d, 3H, Me), 3,75 (m, 1H, CH-Me), 4.1- 4.5 (m, 2H, OCH₂), 4,80 (1H, CH=) 5,0 (1H, CH=) ppm.

## Patentansprüche

1. Verfahren zur Herstellung von Methylen-1,3-dioxolanen der allgemeinen Formel (I), 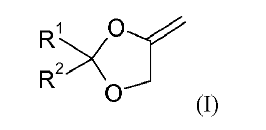 in welcher
R¹ und R² unabhängig voneinander für Wasserstoff, Alkyl, Aryl oder Alkylaryl stehen,
R¹ und R² weiterhin gemeinsam mit dem C-Atom, an das sie geknüpft sind, einen 4- bis 7 gliedrigen, gesättigten, gegebenenfalls substituierten Ring ausbilden können,
**dadurch gekennzeichnet, dass** man Verbindungen der Formel (II), 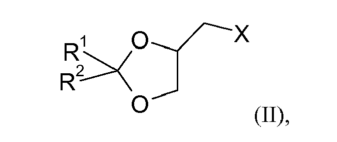 in welcher
R¹, R² die oben angegebenen Bedeutungen haben,
X für Halogen steht,
mit anorganischen Basen in Gegenwart von Polyethylenglykoldimethylethern oder Polyethylenglykoldiethylethern umsetzt.

2. Verfahren zur Herstellung von Verbindungen der Formel (I) gemäß Anspruch 1, **dadurch gekennzeichnet, dass**
R¹ und R² unabhängig voneinander für Wasserstoff, (C₁-C₁₂)-Alkyl, Pentamethylen oder Hexamethylen stehen,
X für Chlor oder Brom steht.

3. Verfahren zur Herstellung von Verbindungen der Formel (I) gemäß einem der Ansprüche 1 oder 2, **dadurch gekennzeichnet, dass** als anorganische Base NaOH, KOH, NaOtBut, KOtBut, NaOMe oder KOMe eingesetzt wird.

4. Verfahren zur Herstellung von Verbindungen der Formel (I) gemäß einem der Ansprüche 1 bis 3, **dadurch gekennzeichnet, dass** Polyethylenglykoldimethylether oder Polyethylenglykoldiethylether mit einer Molmasse von 200 bis 500 eingesetzt werden.

5. Verfahren zur Herstellung von Verbindungen der Formel (I) gemäß einem der Ansprüche 1 bis 4, **dadurch gekennzeichnet, dass** 1 Mol einer Verbindung der allgemeinen Formel (II) mit 0,8 Mol bis 2,5 Mol einer anorganischen Base umgesetzt wird.

6. Verfahren zur Herstellung von Verbindungen der Formel (I) gemäß einem der Ansprüche 1 bis 5, **dadurch gekennzeichnet, dass** das Verfahren in einem Temperaturbereich zwischen 60°C und 150°C durchgeführt wird.

## Claims

1. Method for preparing methylene-1,3-dioxolanes of the general formula (I), 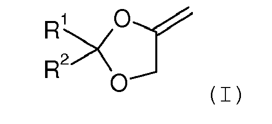 where
R¹ and R² independently of one another are hydrogen, alkyl, aryl or alkylaryl,
R¹ and R² may also form, together with the carbon atom to which they are linked, a 4- to 7-membered, saturated, optionally substituted ring,
**characterized in that** compounds of formula (II), 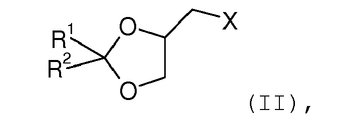 where
R¹, R² have the meanings stated above,
X is halogen,
are reacted with inorganic bases in the presence of polyethylene glycol dimethyl ethers or polyethylene glycol diethyl ethers.

2. Method for preparing compounds of formula (I) according to Claim 1, **characterized in that**
R¹ and R² independently of one another are hydrogen, (C₁-C₁₂)-alkyl, pentamethylene or hexamethylene,
X is chlorine or bromine.

3. Method for preparing compounds of formula (I) according to either Claim 1 or 2, **characterized in that** NaOH, KOH, NaO^{t}Bu, KO^{t}Bu, NaOMe or KOMe is used as inorganic base.

4. Method for preparing compounds of formula (I) according to any of Claims 1 to 3, **characterized in that** polyethylene glycol dimethyl ethers or polyethylene glycol diethyl ethers having a molar mass of 200 to 500 are used.

5. Method for preparing compounds of formula (I) according to any of Claims 1 to 4, **characterized in that** 1 mole of a compound of the general formula (II) is reacted with 0.8 mole to 2.5 moles of an inorganic base.

6. Method for preparing compounds of formula (I) according to any of Claims 1 to 5, **characterized in that** the method is carried out in a temperature range between 60°C and 150°C.

## Revendications

1. Procédé de fabrication de méthylène-1,3-dioxolanes de formule générale (I) 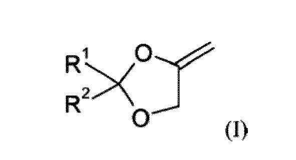 dans laquelle
R¹ et R² représentent indépendamment l'un de l'autre hydrogène, alkyle, aryle ou alkylaryle,
R¹ et R² peuvent également former conjointement avec l'atome C auquel ils sont reliés un cycle de 4 à 7 éléments, saturé, éventuellement substitué,
**caractérisé en ce que** des composés de formule (II) 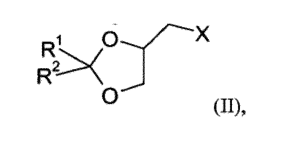 dans laquelle
R¹, R² ont les significations indiquées précédemment,
X représente un halogène,
sont mis en réaction avec des bases inorganiques en présence d'éthers diméthyliques de polyéthylène glycol ou d'éthers diéthyliques de polyéthylène glycol.

2. Procédé de fabrication de composés de formule (I) selon la revendication 1, **caractérisé en ce que**
R¹ et R² représentent indépendamment l'un de l'autre hydrogène, alkyle en (C₁-C₁₂), pentaméthylène ou hexaméthylène,
X représente chlore ou brome.

3. Procédé de fabrication de composés de formule (I) selon l'une quelconque des revendications 1 ou 2, **caractérisé en ce que** NaOH, KOH, NaOtBut, KOtBut, NaOMe ou KOMe est utilisé en tant que base inorganique.

4. Procédé de fabrication de composés de formule (I) selon l'une quelconque des revendications 1 à 3, **caractérisé en ce que** des éthers diméthyliques de polyéthylène glycol ou des éthers diéthyliques de polyéthylène glycol ayant une masse molaire de 200 à 500 sont utilisés.

5. Procédé de fabrication de composés de formule (I) selon l'une quelconque des revendications 1 à 4, **caractérisé en ce que** 1 mole d'un composé de formule générale (II) est mise en réaction avec 0,8 mole à 2,5 moles d'une base inorganique.

6. Procédé de fabrication de composés de formule (I) selon l'une quelconque des revendications 1 à 5, **caractérisé en ce que** le procédé est réalisé dans une plage de température comprise entre 60 °C et 150 °C.
